# EUROPÄISCHE PATENTSCHRIFT

(11) **EP 1 173 396 B1**
(45) Veröffentlichungstag und Bekanntmachung des Hinweises auf die Patenterteilung: **19.05.2004**
(21) Anmeldenummer: 00920541.0
(22) Anmeldetag: 23.03.2000
(51) Int. Cl.: C07B 41/00, C07C 409/04

(54) **SINGLET SAUERSTOFF OXIDATION VON ORGANISCHEN SUBSTRATEN**
SINGLET OXYGEN OXIDATION OF ORGANIC SUBSTRATES
OXYDATION DE SUBSTRATS ORGANIQUES PAR L'OXYGENE SINGULET

(30) Priorität: 26.04.1999 AT 73099
(43) Veröffentlichungstag der Anmeldung: 23.01.2002
(73) Patentinhaber: DSM Fine Chemicals Austria Nfg GmbH & Co KG, 4021 Linz (AT)
(72) Erfinder: AUBRY, Jean-Marie, F-62590 Oignies (FR); RATAJ-NARDELLO, Véronique, F-59650 Villeneuve d'Ascq (FR); ALSTERS, Paul, NL-6224 KZ Maastricht (NL)
(74) Vertreter: Lindinger, Ingrid
(86) Internationale Anmeldenummer: PCT/EP2000/002553
(87) Internationale Veröffentlichungsnummer: WO 2000/064842

(56) Entgegenhaltungen:
- EP-A- 0 288 337
- WO-A-00/61524
- WO-A-97/29066
- US-A- 4 022 841
- D. H. R. BARTON ET AL: "Experiments on the synthesis of tetracycline. Part XIII. Oxidation of ring A model phenols to p-hydroxycyclohexadienones" JOURNAL OF THE CHEMICAL SOCIETY, PERKIN TRANSACTIONS 1, 1975, Seiten 1610-1614, XP002145295 LETCHWORTH GB
- W. P. GRIFFITH: "Lanthanide complexes as oxidation catalysts for alcohols and alkenes" POLYHEDRON, Bd. 15, Nr. 20, 1996, Seiten 3493-3500, XP000914081

## Beschreibung

Die einzige Singlet Sauerstoff Oxidation (¹O₂-Ox), die zur Zeit industriell durchgeführt wird, ist die photochemische ¹O₂-Ox, bei welcher der ¹O₂ auf photochemischen Weg generiert wird. Der Nachteil dieses Verfahrens ist durch die hohen Kosten der benötigten photochemischen Einrichtungen, sowie durch eine beschränkte Lebensdauer gegeben. Die benötigten Lampen degenerieren durch Verschmutzung der Glasoberfläche relativ rasch während der Oxidation. Außerdem eignet sich dieses Verfahren nicht für gefärbte Substrate. Das Verfahren ist eigentlich nur für Feinchemikalien geeignet, die im kleineren Maßstab hergestellt werden. (La Chimica e l'Industria, 1982, Vol. 64, Seite 156) Aus diesem Grund wurde versucht andere Verfahrensvarianten für die ¹O₂-Ox zu finden, die für die ¹O₂-Ox von nicht-wasserlöslichen, hydrophoben organischen Substraten geeignet sind.

In J. Am. Chem. Soc., 1968, **90**, 975 wird beispielsweise die klassische "dark" ¹O₂-Ox beschrieben, bei welcher ¹O₂ nicht photochemisch sondern chemisch generiert wird. Dabei werden hydrophobe Substrate mittels eines Hypochlorit/H₂O₂ - Systems in einem Lösungsmittelgemisch aus Wasser und organischem Lösungsmittel oxidiert. Dieses Verfahren hat jedoch lediglich einige synthetische Anwendungen gefunden, da viele Substrate in dem benötigtem Medium nur schwer löslich sind. Die Einsatzmöglichkeit ist außerdem aufgrund von Nebenreaktionen zwischen Hypochlorit und Substrat oder Lösungsmittel ziemlich eingeschränkt. Außerdem wird in der Gasphase ein großer Teil des ¹O₂ deaktiviert. Weiters ist dieses Verfahren nicht für den industriellen Maßstab geeignet, da es im organischen Medium zur Anlagerung des Hypochlorits an H₂O₂ kommt und ein großer Überschuss an H₂O₂ zur Unterdrückung der Nebenreaktion von Substrat mit Hypochlorit benötigt wird. Ein zusätzlicher Nachteil ergibt sich durch das Anfallen stöchiometrischer Salzmengen.

Eine Variante der "dark" ¹O₂-Ox, die nicht auf Hypochlorit basiert und somit obige Nachteile zum Teil vermeiden soll, ist beispielsweise aus J. Org. Chem., 1989, **54**, 726 oder J. Mol. Cat., 1997, **117**, 439 bekannt, wonach einige wasserlösliche organische Substrate mit H₂O₂ und einem Molybdatkatalysator in Wasser als Lösungsmittel oxidiert werden. Gemäß Membrane Lipid Oxid. Vol. II, 1991, 65 ist die ¹O₂-Ox von wasserunlöslichen, organischen Substraten mit dem Molybdat/ H₂O₂ -System schwierig, da angenommen wurde, dass keines der üblichen Lösungsmittel geeignet ist, die von Molybdat katalysierte Disproportionierung von H₂O₂ in Wasser und ¹O₂ aufrecht zu erhalten. Die Verwendung von Molybdän-Katalysatoren bringt jedoch auch andere Nachteile mit sich. So katalysieren sie neben der H₂O₂ - Disproportionierung auch andere, unerwünschte Oxidationen von manchen Substraten. Allylalkohole können beispielsweise nicht effektiv mit dem Molybdat/ H₂O₂-System peroxidiert werden, da diese Substanzgruppe von Molybdän in Gegenwart von H₂O₂ epoxidiert wird. Ein weiterer Nachteil dieser Katalysatoren ist der relativ geringe pH-Bereich in dem diese funktionieren. Diese Katalysatoren können nur im basischen Bereich zwischen pH 9 und pH 12 eingesetzt werden, die Verwendung dieses Systems ist demnach nicht geeignet für saure Bedingungen.
In J.C.S. Perkin I, 1975, Seiten 160-1614 ist die Singlett-Sauerstoff-Oxidation verschiedener Phenol mit Cer(IV)-Oxid in stöchiometrischen Mengen und H₂O₂(30%ig) in diversen organischen Lösungsmitteln (MeOH, EtOH, t-BuOH) beschrieben, wobei darauf hingewiesen wird, dass die optimale Reaktionstemperatur bei 60 bis 70°C liegt.
WO 97/29066 beschreibt des weiteren die Hydroxylierung von Phenolen bzw. Phenolethern mittels H₂O₂ in Gegenwart eines Seltenen Erdmetallsalzes der Trifluormethansulfonsäure bei Temperaturen von 45 bis 150°C.
Weiters ist aus Polyhedron, Vol 15, Nr. 20, 1996, Seiten 3493 - 3497 bekannt, dass spezielle Lanthaniden Komplexe bei Rückflusstemperatur die Oxidation von Alkoholen und Alkenen katalysieren, während darauf hingewiesen wird, dass "einfache" Salze der Lanthaniden geringen bis keinen katalytischen Effekt aufweisen.

Aufgabe der vorliegenden Erfindung war es demnach, einen Katalysator für die H₂O₂ - Disproportionierung für "dark" ¹O₂-Ox zu finden, der in einem breiten pH-Bereich effektiv ist, insbesondere auch im sauren Bereich und der keine unerwünschten Nebenreaktionen, wie etwa die Epoxidierung von allylischen Alkoholen, neben der H₂O₂ - Disproportionierung katalysiert.

Unerwarteterweise wurde nun gefunden, dass Lanthan als Katalysator sowohl im basischen als auch im sauren Bereich effektiv ist, wobei unerwünschte Nebenreaktionen bei dessen Verwendung nicht oder in deutlich geringerem Ausmaß auftreten. Unerwarteterweise ist dieser Katalysator auch in heterogener Form aktiv, sodass dessen Rückgewinnung aus dem Reaktionsgemisch auf einfachem Weg erfolgen kann.

Gegenstand der vorliegenden Erfindung ist demnach ein Verfahren zur Oxidation von organischen Substraten mittels ¹O₂, das dadurch gekennzeichnet ist, dass organische Substrate aus der Gruppe Olefine, die 1 bis 4 C=C-Doppelbindungen enthalten; C₆-C₂₀ Phenole, Polyalkylbenzole, Polyalkoxybenzolen; polycyclische Aromaten mit 2 bis 4 aromatischen Ringen; C₄-C₂₀ Heterocyclen mit einem O-, N- oder S-Atom im Ring, die unsubstituiert sein können oder ein- oder mehrfach mit Halogenen, Carbonylgruppen, Hydroxylgruppen, C₁-C₆-Alkoxygruppen, C₁-C₆-Alkylgruppen, C₆-C₂₀-Arylgruppen, C₂-C₆-Alkenylgruppen, C₂-C₆-Alkinylgruppen, Carbonsäuregruppen, Estergruppen, Amidgruppen, Nitrogruppen, Silylgruppen, Silyloxygruppen, Sulfongruppen, Sulfoxidgruppen oder mit einem oder mehreren NR¹R²-Resten, in denen R₁ oder R₂ gleich oder verschieden sein können und H; C₁-C₆-Alkyl; Formyl; C₂-C₆-Acyl; C₇-C₂₀-Benzoyl bedeuten, wobei R¹ und R² auch gemeinsam einen Ring bilden können, substituiert sein können, in Wasser, einem organischem Lösungsmittel oder einem Wasser-Lösungsmittel-Gemisch bei einer Reaktionstemperatur zwischen 0 und 50°C in Gegenwart von Lanthan in Form eines Oxides, Nitrates, Carboxylates, Hydroxids, Carbonates, Chlorids, Fluorids oder Sulfates als Katalysators mit 30-70%igem H₂O₂ versetzt werden, worauf anschließend an die katalytische Zersetzung von H₂O₂ zu Wasser und ¹O₂ die Oxidation zu den entsprechenden Oxidationsprodukten erfolgt.

Das erfindungsgemäße Verfahren eignet sich zur Oxidation von organischen Substraten, die mit ¹O₂ reagieren.
Als Substrat können demnach folgende Verbindungen eingesetzt werden: Olefine, die eine oder mehrere, d.h. bis zu zu 4 C=C-Doppelbindungen enthalten; elektronenreiche Aromaten, wie C₆-C₂₀, Phenole, Polyalkylbenzole, Polyalkoxybenzolen; polycyclische Aromaten mit 2 bis 4 aromatischen Ringen; sowie Heterocyclen mit einem O-, N- oder S-Atom im Ring, wie beispielsweise C₄-C₂₀ Furane, C₄-C₂₀ Pyrrole, C₄-C₂₀ Thiophene. Die Substrate können dabei einen oder mehrere Substituenten, wie Halogen (F, Cl, Br, J), Carbonylgruppen, Hydroxylgruppen, C₁-C₆ Alkoxygruppen, C₁-C₆ Alkylgruppen, C₆-C₂₀ Arylgruppen, C₂-C₆ Alkenylgruppen, C₂-C₆ Alkinylgruppen, Carbonsäuregruppen, Estergruppen, Amidgruppen, Nitrogruppen, Silylgruppen, Silyloxygruppen, Sulfongruppen, Sulfoxidgruppen, aufweisen. Weiters können die Substrate substituiert sein mit einem oder mehreren NR¹R²-Resten, in denen R₁ oder R₂ gleich oder verschieden sein können und H; C₁-C₆ Alkyl; Formyl; C₂-C₆ Acyl; C₇-C₂₀ Benzoyl bedeuten, wobei R¹ und R² auch gemeinsam einen Ring bilden können, wie z.B. in einer Phthalimidogruppe.
Beispiele für geeignete Substrate sind: 2-Buten; Isobuten; 2-Methyl-1-buten; 2-Hexen; 1,3-Butadien; 2,3-Dimethylbuten; Δ^{9,10}-Octalin, 2-Phthalimido-4-Methyl-3-penten; 2,3,-Dimethyl-1,3-Butadien; 2,4-Hexadien; 3-Methyl-2-buten-1-ol; 4-Methyl-3-penten-2-ol; 2-Amino-4-methyl-3-penten; 2-Chlor-4-methyl-3-penten; 2-Brom-4-methyl-3-penten; 1-Trimethylsilylcyclohexen; 2,3-Dimethyl-2-butenyl-*para*-tolylsulfon; 2,3-Dimethyl-2-butenyl-*para*-tolylsulfoxid; *N*-Cyclohexenylmorpholin; 2-Methyl-2-norbomen; Terpinolen; α-Pinen; β-Pinen; β-Citronellol; Ocimen; Citronellol; Geraniol; Famesol; Terpinen; Limonen; *trans*-2,3-Dimethylacrylsäure; α-Terpinen; Isopren; Cyclopentadien; 1,4-Diphenylbutadien; 2-Ethoxybutadien; 1,1'-Dicyclohexenyl; Cholesterol; Ergosterolacetat; 5-Chlor-1,3-cyclohexadien; 3-Methyl-2-buten-1-ol; 3,5,5-Trimethylcyclohex-2-en-1-ol; Phenol, 1,2,4-Trimethoxybenzol, 2,3,6-Trimethylphenol, 2,4,6-Trimethylphenol, 1,4-Dimethylnaphthalen, Furan, Furfurylalcohol, Furfural, 2,5-Dimethylfuran, Isobenzofuran, Dibenzylsulfid, (2-Methyl-5-*tert*-butyl)phenylsulfid u.s.w.

Aus den Substraten wird durch die erfindungsgemäße Oxidation das korrespondierende Oxidationsprodukt erhalten. Aus Alkenen, (polycyclischen) Aromaten oder Heteroaromaten werden insbesondere Hydroperoxide oder Peroxide erhalten, die unter den Reaktionsbedingungen zu Alkoholen, Epoxiden, Acetalen oder Carbonylverbindungen, wie Ketone, Aldehyde, Carbonsäuren oder Ester weiter reagieren können, wenn das Hydroperoxid oder das Peroxid nicht stabil ist.

Die erfindungsgemäße Oxidation erfolgt in Wasser oder einem organischem Lösungsmittel.
Geeignete Lösungsmittel sind demnach Wasser, C₁-C₈-Alkohole, wie Methanol, Ethanol, Propanol, i-Propanol, Butanol, i-Butanol; Ethylenglykol, Propylenglykol, Formamid, N-Methylformamid, Dimethylformamid, Sulfolan, Propylencarbonat.
Bevorzugt werden Methanol, Ethanol, Propanol, i-Propanol, Ethylenglykol, Propylenglykol, Formamid, N-Methylformamid oder Dimethylformamid, besonders bevorzugt Methanol, Ethanol, Ethylenglykol, Propylenglykol, Formamid oder Dimethylformamid als Lösungsmittel eingesetzt.
Die Reaktion kann auch in einem Wasser-Lösungsmittel-Gemisch oder in einem Gemisch obiger organischer Lösungsmittel durchgeführt werden.

Dem Lösungsmittel-Substrat-Gemisch wird als heterogener oder homogener anorganischer Katalysator Lanthan zugesetzt. Das Metall kann dabei als Oxid, Nitrat, Carboxylat, Hydroxid, Carbonat, Chlorid, Fluorid oder Sulfat vorliegen. Homogenen, löslichen Formen des Katalysators kann gegebenenfalls ein Hydroxid, beispielsweise NaOH, KOH, u.s.w., zugegeben werden, sodass ein heterogener, aktiver Katalysator erhalten wird.

Die Menge an eingesetztem Katalysator hängt vom eingesetzten Substrat ab und liegt zwischen 1 und 50 mol%, bevorzugt zwischen 5 und 25 mol%

Anschließend erfolgt die Zugabe von 30-70%igem, bevorzugt 40-60%igem H₂O₂. Bevorzugt wird H₂O₂ langsam oder portionsweise dem Reaktionsgemisch aus Lösungsmittel, Substrat und Katalysator zugegeben, wobei das Reaktionsgemisch gerührt wird. Es ist auch möglich zuerst nur einen Teil des H₂O₂ zu einer löslichen Form des gewählten Katalysators, anschließend ein Hydroxid, wie etwa NaOH, KOH u.s.w., und danach die restliche Menge an H₂O₂ zu Der Verbrauch an H₂O₂ ist bei dem erfindungsgemäßen Verfahren vom eingesetzten Substrat abhängig. Für reaktive Substrate werden bevorzugt 2 bis 3 Äquivalente an H₂O₂ benötigt, während weniger reaktive Substrate bevorzugt mit 3 bis 10 Äquivalenten an H₂O₂ umgesetzt werden.

Die Reaktionstemperatur liegt zwischen 0 und 50°C, bevorzugt zwischen 15 und 35°C. Wird die Reaktion in Wasser durchgeführt, so hängt der pH-Wert des Reaktionsgemisches von dem gewählten Substrat ab. Der pH -Wert liegt dabei zwischen 0 und 14, bevorzugt zwischen 4 und 14. Der pH-Wert des Reaktionsgemisches kann dabei wenn notwendig je nach Bedarf mit üblichen basischen oder sauren Zusätzen eingestellt werden.

Der Reaktionsverlauf kann mittels UV-Spektroskopie oder mittels HPLC verfolgt werden. Nach beendeter Reaktion, d.h. nach 1 bis 30 Stunden je nach Reaktionsbedingungen, erfolgt die Aufarbeitung des Reaktionsgemisches und die Abtrennung des Katalysators durch übliche Methoden. Insbesondere bei Verwendung eines heterogenen Katalysators ist die Abtrennung desselben sehr einfach durch simples Abfiltrieren oder zentrifugieren möglich, wodurch das Recyclieren des Katalysators ebenfalls vereinfacht wird.
Das verbleibende Endprodukt kann gegebenenfalls mittels Umkristallisieren, Extrahieren oder Destillation gereinigt werden.

Das erfindungsgemäße Verfahren ermöglicht die Oxidation einer Vielzahl von Verbindungen. Besonders geeignet ist das erfindungsgemäße Verfahren demnach zur Oxidation von ungesättigten organischen Verbindungen, wie Allylalkohole, ungesättigten Aminen, wie Allylaminen, Terpenen, beispielsweise α-Terpinen und Citronellol, aromatischen Polycyclen, Steroiden, Furanen, Cyclopentadienen, Phenolen u.s.w., und allgemein für alle Verbindungen die mit ¹O₂ reagieren.

Durch das erfindungsgemäße Verfahren werden die gewünschten Endprodukte in hohen Ausbeuten von bis zu 100% mit hoher Reinheit erhalten.
Das erfindungsgemäße Verfahren zeichnet sich dabei durch die einfache Prozessführung aus, die sich bestens für den industriellen Maßstab eignet, da sie in einfachen Mehrzweckanlagen und mit einfachen Aufarbeitungsschritten erfolgen kann und für ein breites Spektrum an Substraten angewendet werden kann.

### Beispiel 1: Oxidation von α-Terpinen

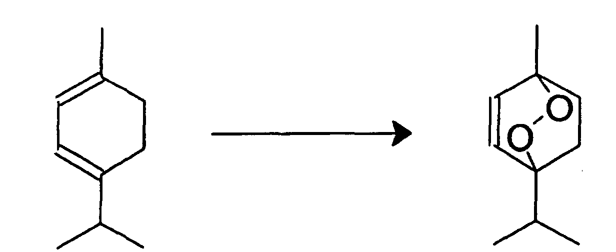

Zu einer Lösung von 325 µl α-Terpinen und 143,6 mg La(NO₃)₃.xH₂O in 4 ml Methanol wurden bei 30°C nacheinander 230 µl H₂O₂ (50%) und 200 µl 5M NaOH zugegeben, worauf sich ein weißer Niederschlag bildete. Nach 3,5 Stunden und nach 21 Stunden wurden dem Gemisch 2 weitere 230 µl -Portionen H₂O₂ (50%) zugesetzt. Nach 24,5 Stunden wurde das Reaktionsgemische zentrifugiert und die Lösung dekantiert vom Katalysator. Das Lösungsmittel wurde evaporiert und der Rückstand gelöst in CDCl₃ und analysiert mit NMR. Das Reaktionsgemisch wurde auch mittels HPLC (MeOH/H₂O 90/10; 260 nm) analysiert. Die Analysen ergaben eine Conversion von 100% und eine Ausbeute von >90% an Ascaridol.

### Beispiel 2: Oxidation von β-Citronellol

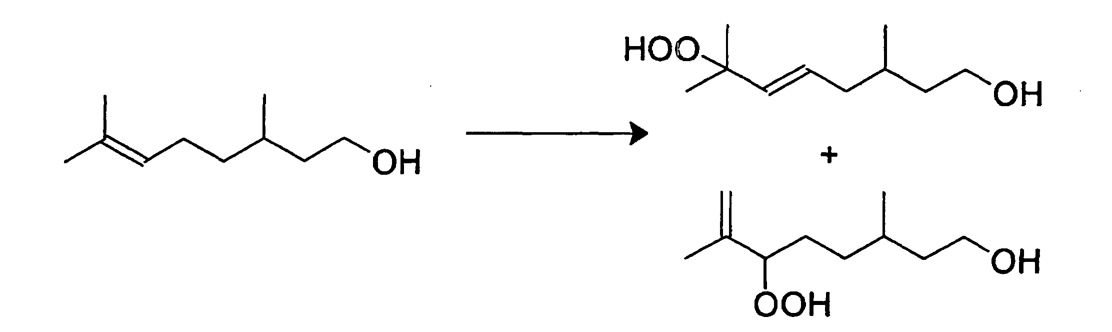

Zu einer Lösung von 391 mg β-Citronellol und 163 mg La(NO₃)₃.xH₂O in 5 ml Methanol wurden bei 25°C nacheinander 300 µl H₂O₂ (50%) und 200 µl 5M NaOH zugegeben, worauf sich ein weißer Niederschlag bildete. Nach 1,3 Stunden und nach 4,0 Stunden wurden dem Gemisch 2 weitere 300 µl -Portionen H₂O₂ (50%) zugesetzt. Nach 5,75 Stunden wurde das Reaktionsgemische mittels HPLC analysiert (MeOH/H₂O 70/30 v/v; 200 nm). Die Analyse ergab eine Conversion von 61% mit einer Ausbeute von sekundärem Hydroperoxid von 24% und einer Ausbeute von tertiärem Hydroperoxid von 37%.

### Beispiel 3: Oxidation von 2-Methyl-2-Butensäure Natrium Salz

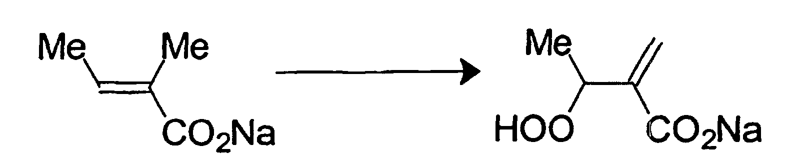

Zu einer Lösung von 50 mg 2-Methyl-2-Butensäure Natrium Salz und 138 mg La(NO₃)₃.xH₂O in 4 ml D₂O wurde bei 25°C 150 µl H₂O₂ (50%) zugegeben, worauf sich ein weißer Niederschlag bildete. Nach 0,75 Stunden und nach 2,0 Stunden wurden dem Gemisch 2 weitere 150 µl -Portionen H₂O₂ (50%) zugesetzt. Nach 3,3 Stunden wurde das Reaktionsgemische mittels NMR analysiert. Die Analyse ergab eine Conversion von 48% mit einer Hydroperoxid Ausbeute von 48%.

### Beispiel 4: Oxidation von 3-Methyl-2-Buten-1-ol

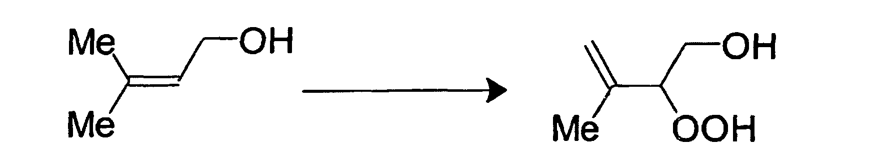

Zu einer Lösung von 215 mg 3-Methyl-2-Buten-1-ol und 163 mg La(NO₃)₃.xH₂O in 5 ml Methanol wurden bei 25°C nacheinander 300 µl H₂O₂ (50%) und 200 µl 5M NaOH zugegeben, worauf sich ein weißer Niederschlag bildete. Nach 1,0 Stunde und nach 4,3 Stunden wurden dem Gemisch 2 weitere 300 µl -Portionen H₂O₂ (50%) zugesetzt. Nach 6,0 Stunden wurde das Reaktionsgemische zentrifugiert und die Lösung dekantiert vom Katalysator. Das Lösungsmittel wurde evaporiert, und der Rückstand gelöst in CDCl₃ und analysiert mit NMR. Die Analyse ergab eine Conversion von 100% mit einer Ausbeute von Hydroperoxid von 70%.

### Beispiel 5: Oxidation von Mesitylol

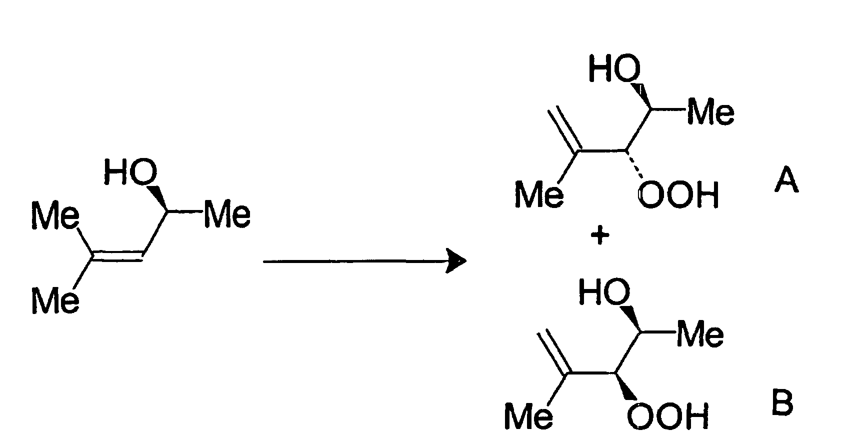

Zu einer Lösung von 257 mg Mesitylol und 163 mg La(NO₃)₃.xH₂O in 5 ml Methanol wurden bei 25°C nacheinander 300 µl H₂O₂ (50%) und 200 µl 5M NaOH zugegeben, worauf sich ein weißer Niederschlag bildete. Nach 1,0 Stunde und nach 4,3 Stunden wurden dem Gemisch 2 weitere 300 µl -Portionen H₂O₂ (50%) zugesetzt. Nach 6,0 Stunden wurde das Reaktionsgemische zentrifugiert und die Lösung dekantiert vom Katalysator. Das Lösungsmittel wurde evaporiert und der Rückstand gelöst in CDCl₃ und analysiert mit NMR. Die Analyse ergab eine Conversion von 100% mit einer Ausbeute von Hydroperoxid A von 62% und einer Ausbeute von Hydroperoxid B von 38%.

### Vergleichsversuch: Oxidation von 3-Methyl-2-Buten-1-ol mit Natrium Molybdat als Katalysator

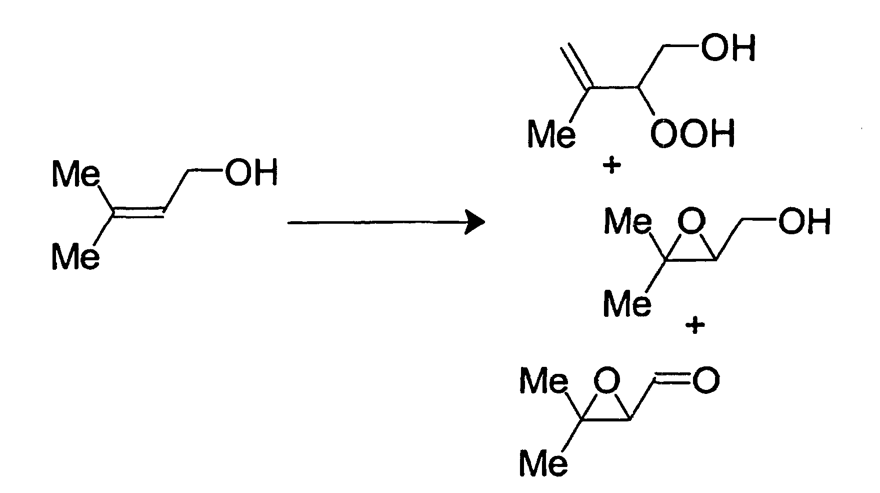

Zu einer Suspension von 100 ml 3-Methyl-2-Buten-1-0l und 121 mg Na₂MoO₄.4H₂O in 5 ml Methanol wurde bei 25°C 100 µl H₂O₂ (50%) zugegeben, worauf eine Klare orange-rote Lösung entstand. Nach 21 Minuten, 42 Minuten, 60 Minuten, 86 Minuten, 120 Minuten, 144 Minuten, 206 Minuten, 224 Minuten, 270 Minuten, 300 Minuten und 333 Minuten wurden dem Gemisch 11 weitere 100 µl -Portionen H₂O₂ (50%) zugesetzt. Nach 6,5 Stunden wurde das Reaktionsgemische zentrifugiert und die Lösung dekantiert vom Katalysator. Das Lösungsmittel wurde evaporiert und der Rückstand gelöst in CDCl₃ und analysiert mit NMR. Die Analyse ergab eine Conversion von 100% mit einer Ausbeute von Hydroperoxid von 31%, von Epoxyalkohol von 59%, und von Epoxyaldehyd von 10%.

### Vergleichsversuch: Photo-oxygenierung von Mesitylol

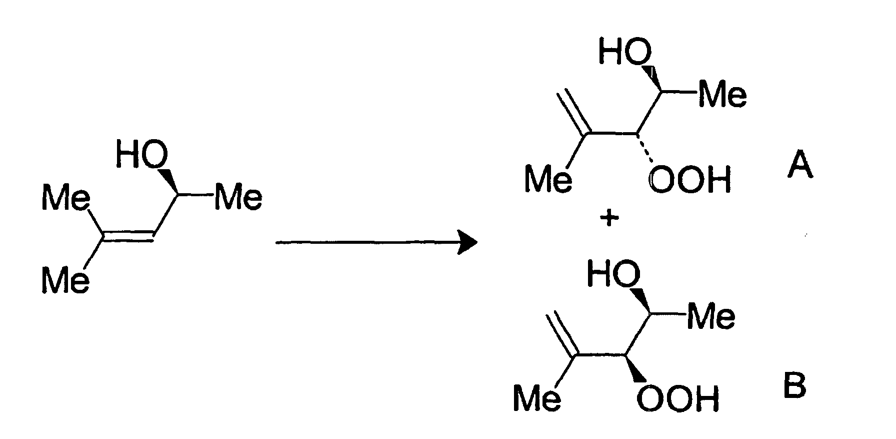

Durch eine Lösung von 40 mg Mesitylol und einer Spur Methylen Blau in 4 ml CD₃OD wurde bei -10°C Sauerstoff geleitet unter Bestrahlung mit einer Na-Larnpe. Nach 3,0 Stunden wurde die Lösung analysiert mit NMR. Die Analyse ergab eine Conversion von 100% mit einer Ausbeute von Hydroperoxid A von 65% und einer Ausbeute von Hydroperoxid B von 35%.

## Patentansprüche

1. Verfahren zur Oxidation von organischen Substraten mittels ¹O₂, **dadurch gekennzeichnet, dass** organische Substrate aus der Gruppe Olefine, die 1 bis 4 C=C-Doppelbindungen enthalten; C₆-C₂₀ Phenole, Polyalkylbenzole, Polyalkoxybenzolen; polycyclische Aromaten mit 2 bis 4 aromatischen Ringen; C₄-C₂₀ Heterocyclen mit einem O-, N- oder S-Atom im Ring, die unsubstituiert sein können oder ein- oder mehrfach mit Halogenen, Carbonylgruppen, Hydroxylgruppen, C₁-C₆-Alkoxygruppen, C₁-C₆-Alkylgruppen, C₆-C₂₀-Arylgruppen, C₂-C₆-Alkenylgruppen, C₂-C₆-Alkinylgruppen, Carbonsäuregruppen, Estergruppen, Amidgruppen, Nitrogruppen, Silylgruppen, Silyloxygruppen, Sulfongruppen, Sulfoxidgruppen oder mit einem oder mehreren NR¹ R²-Resten, in denen R₁ oder R₂ gleich oder verschieden sein können und H; C₁-C₆-Alkyl; Formyl; C₂-C₆-Acyl; C₇-C₂₀-Benzoyl bedeuten, wobei R¹ und R² auch gemeinsam einen Ring bilden können, substituiert sein können, in Wasser, einem organischem Lösungsmittel oder einem Wasser-Lösungsmittel-Gemisch bei einer Reaktionstemperatur zwischen 0 und 50°C in Gegenwart von Lanthan in Form eines Oxides, Nitrates, Carboxylates, Hydroxids, Carbonats, Chlorids, Fluorids oder Sulfates als Katalysators mit 30-70%igem H₂O₂ versetzt werden, worauf anschließend an die katalytische Zersetzung von H₂O₂ zu Wasser und ¹O₂ die Oxidation zu den entsprechenden Oxidationsprodukten erfolgt.

2. Verfahren nach Anspruch 1, **dadurch gekennzeichnet, dass** als Lösungsmittel Wasser, C₁-C₈-Alkohole, Ethylenglykol, Propylenglykol, Formamid, N-Methylformamid, Dimethylformamid, Sulfolan, Propylencarbonat oder Mischungen davon verwendet werden.

3. Verfahren nach Anspruch 2, **dadurch gekennzeichnet, dass** als Lösungsmittel Methanol, Ethanol, Propanol, i-Propanol, Ethylenglykol, Propylenglykol, Formamid, N-Methylformamid oder Dimethylformamid verwendet werden.

4. Verfahren nach Anspruch 1, **dadurch gekennzeichnet, dass** homogene, lösliche Formen des Katalysators durch Zugabe eines Hydroxids in eine heterogene, aktive Form des Katalysators umgewandelt werden.

5. Verfahren nach Anspruch 1, **dadurch gekennzeichnet, dass** in Abhängigkeit vom eingesetzten Substrat 2 bis 10 Äquivalente an H₂O₂ eingesetzt werden.

6. Verfahren nach Anspruch 1, **dadurch gekennzeichnet, dass** in Abhängigkeit von eingesetzten Lösungsmittel, Substrat und Katalysator ein pH-Wert zwischen 0 und 14 eingestellt wird.

## Claims

1. Process for the oxidation of organic substrates by means of ¹O₂, which comprises adding 30-70%. strength H₂O₂ to organic substrates from the group of olefins which contain 1 to 4 C=C double bonds; C₆-C₂₀ phenols, polyalkylbenzenes, polyalkoxybenzenes; polycyclic aromatics having 2 to 4 aromatic rings; C₄-C₂₀ heterocycles with an O, N or S atom in the ring, which may be unsubstituted or may be mono- or polysubstituted by halogens, carbonyl groups, hydroxyl groups, C₁-C₆ alkoxy groups, C₁-C₆ alkyl groups, C₆-C₂₀ aryl groups, C₂-C₆ alkenyl groups, C₂-C₆ alkynyl groups, carboxylic acid groups, ester groups, amide groups, nitro groups, silyl groups, silyloxy groups, sulphone groups, sulphoxide groups or by one or more NR¹R² radicals in which R₁ or R₂ may be identical or different and are H; C₁-C₆ alkyl; formyl; C₂-C₆ acyl; C₇-C₂₀ benzyl, where R¹ and R² may also together form a ring, as catalyst, whereupon, following the catalytic decomposition of H₂O₂ to give water and ¹O₂, oxidation to give the corresponding oxidation products takes place.

2. Process according to Claim 1, **characterized in that** the solvent used is water, C₁-C₈-alcohols, ethylene glycol, propylene glycol, formamide, N-methylformamide, dimethylformamide, sulpholane, propylene carbonate or mixtures thereof.

3. Process according to Claim 2, **characterized in that** the solvent used is methanol, ethanol, propanol, isopropanol, ethylene glycol, propylene glycol, formamide, N-methylformamide or dimethylformamide.

4. Process according to Claim 1, **characterized in that** homogeneous, soluble forms of the catalyst are converted into a heterogeneous, active form of the catalyst by adding a hydroxide.

5. Process according to Claim 1, **characterized in that** 2 to 10 equivalents of H₂O₂ are used depending on the substrate used.

6. Process according to Claim 1, **characterized in that** a pH between 0 and 14 is set depending on the solvent, substrate and catalyst used.

## Revendications

1. Procédé pour l'oxydation de substrats organiques à l'aide du ¹O₂, **caractérisé en ce que** le substrat organique est sélectionné dans le groupe des oléfines, qui contiennent 1 à 4 doubles liaisons C=C ; des phénols en C₆-C₂₀, des polyalkylbenzènes en C₆-C₂₀, des polyalcoxybenzènes en C₆-C₂₀; des aromatiques polycycliques ayant 2 à 4 cycles aromatiques ; des hétérocycles en C₄-C₂₀ avec un atome O, N ou S dans le cycle, qui peuvent être non substitués ou substitués une ou plusieurs fois, par des halogènes, des radicaux carbonyle, des radicaux hydroxyle, des radicaux alcoxy en C₁-C₆, des radicaux alkyle en C₁-C₆, des radicaux alryle en C₆-C₂₀, des radicaux alcényle en C₂-C₆, des radicaux alcynyle en C₂-C₆, des radicaux acide carboxylique, des radicaux ester, des radicaux amide, des radicaux nitro, des radicaux silyle, des radicaux siloxy, des radicaux sulfone, des radicaux sulfoxyde ou avec un ou plusieurs groupement NR¹R², dans lesquels R¹ ou R² peuvent être identiques ou différents et représentent H, alkyle en C₁-C₆, formyle, acyle en C₂-C₆, benzoyle en C₇-C₂₀, où R¹ et R² peuvent également former ensemble, un cycle, est mis à réagir dans l'eau, un solvant organique ou un mélange eau-solvant organique, à une température de réaction allant de 0 à 50°C en présence du lanthane sous la forme d'un oxyde, d'un nitrate, d'un carboxylate, d'un hydroxyde, d'un carbonate, d'un chlorure, d'un fluorure ou d'un sulfate, comme catalyseur avec H₂O₂ à 30-70%, où suite à la décomposition catalytique du H₂O₂ en eau et ¹O₂, l'oxydation est réalisée en les produits d'oxydation appropriés.

2. Procédé selon la revendication 1, **caractérisé en ce que** l'on utilise comme solvant, l'eau, des alcools en C₁-C₈, l'éthylèneglycol, le propylèneglycol, le formamide, le N-méthylformamide, le diméthylformamide, le sulfolanne, le carbonate de propylène ou leurs mélanges.

3. Procédé selon la revendication 2, **caractérisé en ce que** l'on utilise comme solvant, le méthanol, l'éthanol, le propanol, le i-propanol, l'éthylèneglycol, le propylèneglycol, le formamide, le N-méthylformamide ou le diméthylformamide.

4. Procédé selon la revendication 1, **caractérisé en ce que** les formes homogènes, solubles du catalyseur sont converties en une forme hétérogène, active du catalyseur par addition d'un hydroxyde.

5. Procédé selon la revendication 1, **caractérisé en ce que** l'on met en oeuvre 2 à 10 équivalents de H₂O₂ en fonction du substrat mis en oeuvre.

6. Procédé selon la revendication 1, **caractérisé en ce que** l'on ajuste le pH entre 0 et 14, en fonction du solvant, du substrat et du catalyseur mis en oeuvre.
